# FASCICULE DE BREVET EUROPEEN

(11) **EP 0 347 282 B1**
(45) Date de publication et mention de la délivrance du brevet: **21.12.1994**
(21) Numéro de dépôt: 89401551.0
(22) Date de dépôt: 06.06.1989
(51) Int. Cl.: A61M 16/00

(54) **Appareil de ventilation spontanée avec assistance respiratoire variable**
Gerät für die Spontanbeatmung mit variabler Atmungshilfe
Spontaneous ventilation apparatus with variable respiratory assistance

(30) Priorité: 16.06.1988 FR 8808064
(43) Date de publication de la demande: 20.12.1989
(73) Titulaire: L'AIR LIQUIDE, SOCIETE ANONYME POUR L'ETUDE ET L'EXPLOITATION DES PROCEDES GEORGES CLAUDE, 75321 Paris Cédex 07 (FR)
(72) Inventeur: Bourdon, Guy, F-78150 Le Chesnay (FR); Griffe, Olivier, F-34000 Montpellier (FR); Robert, Dominique, F-69410 Champagne-au-Mont-d'Or (FR)
(74) Mandataire: Le Moenner, Gabriel

(56) Documents cités:
- US-A- 4 584 996
- US-A- 4 612 928

## Description

L'invention a pour objet un appareil de ventilation méchanique destiné à être mis en oeuvre pour des prothèses respiratoires appelées ventilateurs.

L'invention concerne plus particulièrement un appareil de ventilation spontanée asservie permettant d'adapter en permanence l'assistance apportée par le ventilateur en fonction de l'état du patient, notamment en vue de faciliter le sevrage.

Les appareils de ventilation spontanée asservie connus sont de deux types :
Un premier type d'appareils de ventilation mécanique (dit MV "mechanical ventilation") est asservi à une concentration en gaz carbonique, (connus sous l'abréviation MV - CO₂), consistant à alterner des phases de ventilation spontanée et de ventilation contrôlée en fonction de la concentration en CO₂ fin d'expiration (système dit "Fraction of end tidal" ou FetCO₂"). Aussi longtemps que la "FetCO₂" est comprise à l'intérieur d'une gamme déterminée de valeurs l'appareil fonctionne en ventilation spontanée. Au contraire dès que la "FetCO₂" sort de cette fourchette, l'appareil passe en ventilation contrôlée jusqu'à ce que la "FetCO₂" revienne à l'intérieur d'une seconde gamme de valeurs, situation où l'appareil repasse alors en ventilation spontanée.

Un second appareil de ventilation spontanée asservie est du type à ventilation imposée variable "VIV" (appelée également "Mandatory Minute Volume" MMV) qui détermine un seuil de volume minute expiré que le patient doit dépasser en ventilation spontanée. Si le volume minute expiré est supérieur à ce seuil, l'assistance fournie par l'appareil est progressivement diminuée jusqu'à devenir éventuellement nulle. Au contraire, si le volume minute expiré est inférieur à ce seuil, l'assistance fournie par l'appareil est augmentée jusqu'à ce que le volume minute expiré dépasse le seuil.

Dans les réalisations connues (Voir US-A-4 612 928 ou US-A-4 584 996), ces appareils assurent cette assistance soit par insufflation de volumes prédéterminés avec une fréquence variable, soit par une aide inspiratoire variable.

Les deux modes respiratoires présentent chacun certains inconvénients :
La MV-CO₂ (consiste à asservir l'assistance du respirateur à la concentration en CO₂ en fin d'expiration ("FetCO₂). Or les variations de la "FetCO₂" ne sont pas toujours correlées avec les variations de la Pa CO₂ (pression partielle du CO₂ dans le sang artériel) qui est le reflet de l'état du patient. De plus, ce mode nécessite l'utilisation d'un analyseur de CO₂ qui reste un appareillage relativement coûteux. C'est probablement pour cette raison que la "MV-CO₂" n'a pas connu de développement commercial important.

En ce qui concerne la "VIV", fondée sur le maintien d'une ventilation minimum, il s'avère qu'une augmentation de ventilation n'est pas forcément une indication d'une évolution favorable de l'état du patient. En effet, l'augmentation de ventilation peut être notamment consécutive à un stress subi par le patient (aspiration trachéale pour désencombrement, piqûre, ...) et traduit plutôt une augmentation de travail. Dans ce cas, l'appareil fonctionnant en mode VIV va diminuer l'assistance apportée au patient, qui en a en réalité encore plus besoin.

Pour pallier à cet inconvénient, on est obligé d'adjoindre à la VIV des alarmes fondées sur le volume courant ou la fréquence, permettant d'interrompre le processus.

Compte tenu de ces remarques, le problème posé est de rechercher un appareil mettant en oeuvre un paramètre plus significatif que les précédents de l'évolution de l'état d'un patient, et facilement accessible.

C'est après une réflexion approfondie que l'on a retenu, à titre de paramètre significatif de l'état respiratoire, la fréquence respiratoire.

Ce choix est motivé pour les raisons suivantes :
Lorsqu'un patient est en ventilation spontanée, un besoin supplémentaire en oxygène va se traduire par une augmentation de la fréquence respiratoire, représentant le supplément de travail fourni pour obtenir plus d'oxygène. De même un besoin moindre en oxygène va se traduire par un ralentissement de la fréquence respiratoire, représentant l'économie du travail à fournir.

Si l'on considère que, à chaque état du patient, correspond une quantité de travail à fournir optimale pour laquelle la fatique n'apparaît pas, il est alors intéressant d'augmenter l'assistance apportée au patient lorsque sa fréquence respiratoire augmente, et de diminuer cette assistance lorsque sa fréquence respiratoire dimiune.

A titre de solution pour obtenir cette action, l'invention vise un appareil qui tend ainsi à maintenir la fréquence respiratoire constante.

L'asservissement fonctionne de la façon suivante :
Lorsque la fréquence respiratoire se situe dans une plage déterminée, l'assistance apportée par l'appareil au patient est maintenue constante.

Lorsque la fréquence respiratoire devient supérieure à la plage déterminée, l'assistance respiratoire est progressivement augmentée. L'augmentation d'assistance respiratoire fournissant le supplément de travail dont a besoin le patient, celui-ci va ralentir sa fréquence respiratoire jusqu'à ce qu'elle revienne à la plage de fréquence choisie.

Lorsque la fréquence respiratoire est inférieure à cette plage, l'assistance respiratoire est progressivement diminuée. La diminution de l'assistance respiratoire compensant l'économie de travail du patient, celui-ci va augmenter sa fréquence jusqu'à ce qu'elle revienne dans la plage prédéterminée, également appelée "objectif fréquence".

Pour réaliser ces objectifs, l'invention propose un appareil de ventilation avec assistance respiratoire variable, du genre comprenant un dispositif de délivrance de débit respiratoire à pression positive constante, des moyens de réglage dudit dispositif de délivrance de débit respiratoire et cet appareil se caractérise en ce que le moyen de réglage du dispositif de délivrance de débit respiratoire est lui-même asservi à un signal de sortie d'un moyen de comparaison entre un signal de référence préréglé et un signal de fréquence respiratoire élaboré à partir d'un moyen détecteur de début de cycle respiratoire, qui est traité par un moyen calculateur de fréquence respiratoire, l'asservissement du moyen de réglage du dispositif de délivrance de débit respiratoire étant conçu pour accroître la pression positive si la fréquence mesurée s'écarte par excès de la fréquence de référence préréglée et pour réduire la pression positive si la fréquence mesurée s'écarte par défaut de ladite fréquence de référence préréglée.

On voit que cet appareil permet d'éviter toute fatique inutile du patient en compensant tout surcroît de travail et augmente progressivement son autonomie lorsque son état s'améliore.

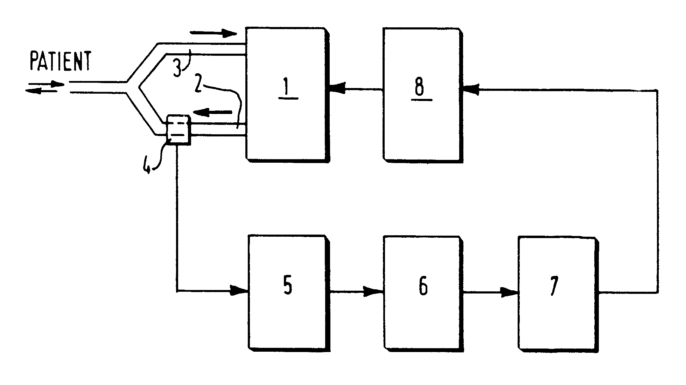

Un appareil conforme à l'invention est maintenant décrit schématiquement en référence au dessin annexé.

L'appareil comprend un dispositif 1 de fourniture d'un débit respiratoire du type à ventilation spontanée avec aide inspiratoire, c'est-à-dire avec maintien d'une pression positive constante pendant les phases d'insufflation. Cet appareil 1 peut être un générateur de débit du type décrit dans la demande de brevet européen n° 272.185 publiée le 22 juin 1988 au nom de la présente demanderesse. L'appareil est raccordé à une source de gaz respiratoire sous pression non représentée et comporte un conduit d'insufflation 2 et un conduit d'expiration 3.

Sur le conduit d'insufflation 2 est monté un détecteur 4 de début de cycle respiratoire, qui est de tout type approprié sensible à un début d'inspiration. A titre d'exemple, on a utilisé un détecteur de la firme SENSYN, type SX01, tel que mentionné et décrit dans le catalogue 1986 de cette firme intitulé "Pressure Sensor Handbook de la page A-15 à A-25. Le signal détecté en 4 est transféré vers un calculateur 5 qui élabore un signal représentatif de la fréquence respiratoire du patient. Ce signal note en fait les tendances d'évolution de la fréquence respiratoire.

Le signal ainsi élaboré en 5 est transmis à un moyen comparateur 6 qui compare ce signal élaboré avec un signal de référence préréglé, ou plutôt à une plage de référence préréglée.

Le signal différentiel de sortie de comparateur 6 est appliqué à un calculateur de niveau d'aide inspiratoire 7 de la façon suivante :
- si le signal différentiel est nul, le calculateur 7 assure le maintien de l'aide inspiratoire ;
- si le signal différentiel est positif (+1), le calculateur 7 accroît le niveau d'aide inspiratoire ;
- si le signal différentiel est négatif (-1), le calculateur 7 réduit le niveau d'aide inspiratoire.

L'action ainsi décrite du calculateur 7 intervient sur un moyen de commande 8 agissant sur le dispositif de débit respiratoire 1 pour en modifier, si besoin est, le niveau de pression de sortie.

Dans l'exemple de réalisation décrit ci-après, l'assistance est réalisée par aide inspiratoire dite "AI", technique connue consistant à appliquer une pression positive constante pendant la phase inspiratoire des cycles spontanés.

Le médecin règle sur le ventilateur la valeur de la plage de fréquence "OF" retenue. A cette valeur peut être associée automatiquement une fourchette, par exemple + 3 cycles/min, dans laquelle le niveau de l'aide inspiratoire "AI" est maintenu constant.

Le calculateur 5 examine ensuite tous les "n" cycles la valeur moyenne de la fréquence "fn" sur les "n" derniers cycles.

Si fn > OF+3, l'aide inspiratoire est augmentée d'une pression égale à 1 cm d'eau ;
Si OF-3 < fn < OF+3, l'aide inspiratoire n'est pas modifiée ;
Si fn < OF-3, l'aide inspiratoire est diminuée d'une pression égale à 1 cm d'eau.

L'appareil comporte en outre une alarme d'aide respiratoire maximum "AI maxi", qui lorsqu'elle est atteinte, se déclenche et interdit toute augmentation du niveau d'AI au delà de cette limite.

L'appareil peut aussi comporter une alarme de fréquence maximum.

On a représenté ici des moyens calculateurs comparateur et de commande schématiquement distincts. Il est bien évident que ces différentes fonctions peuvent être rempliées par des moyens automatiquement programmés.

## Revendications

1. Appareil de ventilation mécanique spontanée avec assistance respiratoire variable, du genre comprenant un dispositif de ventilation (1) délivrant, dans un conduit d'insufflation (2), un débit respiratoire à un niveau de pression positive déterminée et comprenant des moyens de réglage du niveau de pression positive, caractérisé en ce qu'il comporte, de façon connue en soi, un détecteur de pression (4) sensible à la pression dans le conduit d'insufflation (2), couplé à un dispositif de commande (5, 6, 7) fournissant un signal de commande de ventilation, et en ce que le dispositif de commande élabore (7) un signal de commande représentatif d'une variation de la fréquence respiratoire détectée par rapport à une fréquence respiratoire de référence prédéterminée et transmis aux moyens de réglage du dispositif de ventilation (1) pour modifier le niveau de pression positive délivrée afin de maintenir la fréquence respiratoire sensiblement constante à la valeur de référence prédéterminée.

2. Appareil selon la revendication 1, caractérisé en ce que les moyens de réglage modifient le niveau de pression positive délivré proportionnellement à la variation de fréquence respiratoire détectée.

3. Appareil selon la revendication 2, caractérisé en ce que les moyens de réglage sont actionnés par le signal de commande lorsque la variation de fréquence détectée est supérieure à un seuil de variation déterminé.

## Claims

1. Spontaneous mechanical ventilation apparatus with variable respiratory assistance, of the type comprising a ventilation device (1) delivering, in an insufflation tube (2), a respiratory flow at a given positive pressure level and comprising means for regulating the positive pressure level, characterised in that it includes, in a manner known per se, a pressure detector (4) sensitive to the pressure in the insufflation tube (2), coupled to a control device (5, 6, 7) providing a ventilation control signal, and in that the control device produces (7) a control signal representing any variation in the detected respiratory frequency compared with a predetermined reference respiratory frequency and transmitted to the means for regulating the ventilation device (1) in order to modify the positive pressure level delivered so as to keep the respiratory frequency substantially constant at the predetermined reference value.

2. Apparatus according to Claim 1, characterised in that the regulation means modify the positive pressure level delivered in proportion to the detected respiratory frequency variation.

3. Apparatus according to Claim 2, characterised in that the regulation means are actuated by the control signal when the variation in frequency detected is greater than a given variation threshold.

## Patentansprüche

1. Vorrichtung zur spontanen mechanischen Beatmung mit variabler Atmungsunterstützung, mit einer Belüftungsvorrichtung (1), die in einer Einblasleitung (2) einen Atmungsdurchsatz auf einem bestimmten positiven Druckniveau liefert und eine Einrichtung zum Regeln des positiven Druckniveaus umfaßt, dadurch gekennzeichnet, daß sie in an sich bekannter Weise einen auf den Druck in der Einblasleitung (2) ansprechenden Druckdetektor (4) umfaßt, der mit einer Steuervorrichtung (5, 6, 7) verbunden ist, die ein Beatmungssteuersignal liefert, und daß die Steuervorrichtung ein Steuersignal erzeugt (7), das für eine in bezug auf eine vorbestimmte Bezugsatmungsfrequenz ermittelte Änderung der ermittelten Atmungsfrequenz repräsentativ ist und an eine Regeleinrichtung der Beatmungsvorrichtung (1) zur Änderung des gegebenen positiven Druckniveaus überträgt, um die Atmungsfrequenz im wesentlichen konstant auf dem Wert der vorbestimmten Bezugsfrequenz zu halten.

2. Vorrichtung nach Anspruch 1, dadurch gekennzeichnet, daß die Regeleinrichtung das gegebene positive Druckniveau proportional zur Änderung der ermittelten Atmungsfrequenz modifiziert.

3. Vorrichtung nach Anspruch 2, dadurch gekennzeichnet, daß die Regeleinrichtungen von dem Steuersignal betätigt wird, wenn die ermittelte Frequenzänderung größer als ein vorbestimmter Schwellwert der Änderung ist.
